# EUROPÄISCHE PATENTSCHRIFT

(11) **EP 3 050 581 B1**
(45) Veröffentlichungstag und Bekanntmachung des Hinweises auf die Patenterteilung: **01.08.2018**
(21) Anmeldenummer: 16150716.5
(22) Anmeldetag: 11.01.2016
(51) Int. Cl.: A61L 29/16, A61L 31/16

(54) **MEDIZINPRODUKT MIT WIRKSTOFFBESCHICHTUNG UND VERFAHREN ZU DESSEN HERSTELLUNG**
MEDICAL PRODUCT WITH AGENT COATING AND METHOD FOR PRODUCING THE SAME
PRODUIT MÉDICAL ENROBÉ D'UN AGENT ACTIF ET SON PROCÉDÉ DE FABRICATION

(30) Priorität: 30.01.2015 DE 102015101380
(43) Veröffentlichungstag der Anmeldung: 03.08.2016
(73) Patentinhaber: DOT GmbH, 18059 Rostock (DE)
(72) Erfinder: NEUMANN, Hans-Georg, 18146 Rostock (DE); BUHRMEISTER, Mischa, 18059 Papendorf (DE)
(74) Vertreter: Prinz & Partner mbB

(56) Entgegenhaltungen:
- WO-A2-2006/063021
- US-A1- 2008 268 018

## Beschreibung

Die Erfindung betrifft ein Medizinprodukt mit Wirkstoffbeschichtung, insbesondere zur Behandlung von Gefäßerkrankungen, und ein Verfahren zu dessen Herstellung.

Krankhaft verengte Blutgefäße (Stenosen) können durch Implantation von Gefäßstützen wie Stents und/oder durch Aufweiten der Gefäßwand mit einem Ballonkatheter behandelt werden. Der an einem Gefäßkatheter angebrachte Ballon wird über eine Arterie, beispielsweise die Beinschlagader, eingeführt und unter Röntgenkontrolle zur verengten Gefäßstelle vorgeschoben. Dort wird der Ballon langsam unter Druck entfaltet. Die Verengung wird dadurch erweitert und ein ungestörter Blutfluss ermöglicht. Um eine erneute Verengung zu verhindern, kann zusätzlich ein Stent implantiert werden. Abhängig vom Stenoseort, der Gefäßgröße und Vorerkrankungen können mit Medikamenten beschichtete Stents verwendet werden.

Bei einigen Patienten kommt es nach dem Aufweiten der Stenosen schon nach wenigen Monaten zu einer erneuten Verengung (Restenose). Die Restenose kann auf eine übermäßige Proliferation insbesondere der glatten Muskelzellen zurückgeführt werden, die durch Verletzungen aufgrund der gewaltsamen Aufweitung der Gefäßwände oder die Implantation des Stents ausgelöst wird. Nach Abheilung der Verletzungen kommt die Proliferation nicht sofort zum Stillstand und führt so häufig zu einer Restenose. Zur Verhinderung einer Restenose wurden mit Medikamenten beschichtete Ballonkatheter, sogenannte "Drug Eluting Ballons", entwickelt. Ein häufig verwendeter Wirkstoff mit anti-proliferativer Wirkung ist Paclitaxel.

Mit Medikamenten beschichtete Ballonkatheter und Stents können außerdem dazu genutzt werden, eine lokale Arzneimittelgabe an die Gefäßwand zu bewirken, ohne dass ein Aufweiten der Gefäße erfolgt, beispielsweise im Falle einer Behandlung von Veränderungen der Gefäßwand, die nicht mit einer Stenose verbunden sind (z. B. vulnerable Plaques, aufgelagerte Thromben), oder bei der Behandlung von Gefäßen mit mechanischen Mitteln oder mit thermischen Verfahren.

Aus der WO 2005/089855 A1 sind mit Medikamenten beschichtete Ballonkatheter und Stents bekannt. Zum Auftragen der Beschichtung kann ein
Lösungsmittel wie Ethanol oder ein Lösungsmittelgemisch verwendet werden. Die Beschichtung kann durch Tauchbeschichten, Sprühbeschichten oder Tintendruckverfahren wie Jetten auf die Stentoberfläche oder den Ballonkatheter aufgebracht werden.

Die DE 20 2010 017 248 U1 offenbart einen Ballonkatheter, der eine Beschichtung aus Paclitaxel und Schellack aufweist. Die Verwendung von Schellack als Bindemittel soll während der kurzen Kontaktzeit des Ballonkatheters mit der Gefäßwand eine schnelle Freisetzung des Wirkstoffs Paclitaxel begünstigen. Als Lösungsmittel für die Beschichtung mit Paclitaxel können Aceton, Ethylacetat, Ethanol, Methanol, Dimethylsulfoxid, Tetrahydrofuran, Chloroform und Methylenchlorid dienen.

Die DE 10 2007 003 184 A1 beschreibt die Beschichtung eines dilatierbaren Katheterballons unter Verwendung einer Lösung von Paclitaxel in Dimethylsulfoxid und Ausfällen des Paclitaxels aus der Lösung durch Zugabe von Wasser.

Die WO 2006/060321 offenbart ein Verfahren zur Beschichtung von Stents, wobei zur Beschichtung eine Lösung verwendet wird, die ein Polymer und einen Wirkstoff enthält. Bevorzugte Lösungsmittel sind Gemische aus THF und Toluol sowie wahlweise Isopropanol.

Die US 2006/0268018 A offenbart ein Verfahren zur Kristallisation eines Wirkstoffs in einer Implantatbeschichtung, wobei das Implantat mit einer Zusammensetzung beschichtet wird, die mindestens ein Polymer und mindestens einen kristallisierbaren Wirkstoff enthält, die in einem Lösungsmittel oder Lösungsmittelgemisch gelöst sind, zusammen mit im Lösungsmittel suspendierten, nichtlöslichen Keimbildungsteilchen.

Die bekannten Verfahren zur Herstellung beschichteter Stents oder Ballonkatheter durch Sprühbeschichten und Tauchbeschichten aus flüchtigen organischen Lösungsmitteln führen üblicherweise zu leicht löslichen Wirkstoffbeschichtungen mit einer glatten, amorphen Struktur, die eine geringe Haftung an der Oberfläche des Stents oder Ballons zeigen und leicht an die Gefäßwand abgegeben werden.

Die glatte, amorphe Struktur der Beschichtung soll verhindern, dass sich der Wirkstoff schon beim Einführen des Stents oder Ballons in die Blutgefäße ablöst und eine unzureichende Wirkstoffmenge an die Gefäßwand am Stenoseort abgegeben wird. Wünschenswert wäre jedoch auch eine Verlängerung der Freisetzungsrate des Wirkstoffs am Stenoseort, um eine Restenose sicher zu verhindern.

Der Erfindung liegt daher die Aufgabe zugrunde, ein Verfahren zur Herstellung von mit Medikamenten beschichteten Medizinprodukten anzugeben, das eine möglichst gut haftende Wirkstoffbeschichtung erzielt und eine Verlängerung der Wirkstoffabgabe an die Gefäßwand begünstigt.

Erfindungsgemäß wird diese Aufgabe durch ein Verfahren nach Anspruch 1 gelöst.

Vorteilhafte Ausführungsformen der Erfindung sind in den Unteransprüchen angegeben, die wahlweise miteinander kombiniert werden können.

Das erfindungsgemäße Verfahren ist auf alle Medizinprodukte anwendbar, die zur Behandlung von Gefäßerkrankungen durch Wirkstoffabgabe an die Gefäßwand eingesetzt werden. Solche Medizinprodukte umfassen in der Regel ein Substrat mit einer Oberfläche und eine auf der Oberfläche aufgebrachte Beschichtung, die einen Wirkstoff enthält.

Erfindungsgemäß umfasst das Verfahren zur Herstellung des Medizinprodukts mit Wirkstoffbeschichtung die folgenden Schritte:
Bereitstellen des Substrats mit der Oberfläche;
Bereitstellen einer Beschichtungslösung des Wirkstoffs in einem Gemisch von wenigstens einem ersten und einem zweiten Lösungsmittel, wobei der Wirkstoff sowohl in dem ersten als auch dem zweiten Lösungsmittel löslich ist;
Auftragen der Beschichtungslösung auf wenigstens einen Teil der Oberfläche des Substrats; und
Entfernen der Lösungsmittel unter Bildung einer den Wirkstoff enthaltenden Beschichtung auf der Oberfläche;
wobei das erste Lösungsmittel einen Siedepunkt von unter 100 °C und das zweite Lösungsmittel einen Siedepunkt von über 130 °C aufweist und die Lösung den Wirkstoff und das zweite Lösungsmittel in einem Anteil enthält, so dass der Anteil des Wirkstoffs über der Löslichkeitsgrenze des Wirkstoffs in dem zweiten Lösungsmittel liegt, und wobei das Entfernen der Lösungsmittel so gesteuert wird, dass zunächst im Wesentlichen das erste Lösungsmittel und danach das zweite Lösungsmittel entfernt wird, wobei nach Entfernen der Lösungsmittel eine Beschichtung erhalten wird, die den Wirkstoff in kristalliner Form enthält.

Durch die Verwendung eines Gemischs von Lösungsmitteln mit unterschiedlichem Siedepunkt oder Dampfdruck lässt sich der Kristallisationsprozess des darin gelösten Wirkstoffs in beliebiger Weise steuern und die Kristallmorphologie des Wirkstoffs in gewünschter Weise einstellen. Da der Wirkstoff in allen Komponenten des Lösungsmittelgemischs löslich ist, wird auch bei Verwendung des Lösungsmittelgemischs eine klare Beschichtungslösung erhalten. Damit kann eine homogene Verteilung des Wirkstoffs auf der Substratoberfläche erzielt werden.

Da die im Gemisch verwendeten Lösungsmittel einen verschiedenen Siedepunkt und damit einen unterschiedlichen Dampfdruck aufweisen, werden sie beim Entfernen der Lösungsmittel unterschiedlich schnell aus der Lösung verdampft. Das erste Lösungsmittel mit niedrigem Siedepunkt und hohem Dampfdruck verflüchtigt sich dabei schneller als das zweite Lösungsmittel, das einen um eine Größenordnung niedrigeren Dampfdruck als das erste Lösungsmittel aufweisen kann.

Das erste, leichtflüchtige Lösungsmittel stellt dabei die vollständige Löslichkeit des Wirkstoffs in der Beschichtungslösung sicher und verdampft nach dem Auftragen der Wirkstofflösung auf die Substratoberfläche innerhalb kurzer Zeit rückstandsfrei aus der Lösung. Da der Anteil des zweiten, schwerflüchtigen Lösungsmittels unter der Löslichkeitsgrenze des Wirkstoffs liegt, bleibt eine amorphe, halbfeste Aufschlämmung des Wirkstoffs im zweiten Lösungsmittel zurück, die aufgrund ihrer hohen Viskosität ortsfest auf der Substratoberfläche haftet. Damit ist gewährleistet, dass die Beschichtung während der Entfernung des zweiten Lösungsmittels nicht mehr verläuft und homogen auf der Substratoberfläche erhalten bleibt.

Über den Dampfdruck des zweiten, schwerflüchtigen Lösungsmittels und das Verhältnis des Wirkstoffanteils zum Anteil des zweiten Lösungsmittels in der Lösung kann die Kristallmorphologie des Wirkstoffs in der nach Entfernen der Lösungsmittel erhaltenen Beschichtung eingestellt werden. Je länger der Wirkstoff in Kontakt mit dem zweiten Lösungsmittel steht, desto mehr Zeit wird für die Kristallbildung und das Kristallwachstum des Wirkstoffs zur Verfügung stehen. Während das zweite Lösungsmittel langsam aus der Beschichtung entfernt wird, tritt somit eine Umkristallisation des Wirkstoffs von einer amorphen Struktur in eine stabile Kristallphase ein, die fest auf der Substratoberfläche haftet.

Der in kristalliner Form in der Beschichtung vorliegende Wirkstoff kann dann am Stenoseort in das umliegende Gewebe eindringen. Die schwerlöslichen Wirkstoffkristalle werden dort weniger schnell abgebaut als leicht lösliche amorphe Wirkstoffformen und können somit länger auf die Zellen in der Gefäßwand einwirken. Damit wird eine längere Hemmung der Zellproliferation erreicht.

Gemäß einer bevorzugten Ausführungsform ist das Substrat der Ballon eines Ballonkatheters oder eine Gefäßstütze, beispielsweise ein Stent. Das Medizinprodukt kann den Ballonkatheter, den Stent oder eine Kombination aus Ballonkatheter und Stent umfassen.

Als Wirkstoff wird im Sinne der Erfindung eine pharmakologisch wirksame Substanz verstanden, insbesondere ein Arzneistoff. Der in dem Lösungsmittelgemisch gelöste Wirkstoff ist vorzugsweise eine anti-proliferative, anti-inflammatorische, anti-phlogistische, anti-hyperplastische, antineoplastische, anti-mitotische, zytostatische, zytotoxische, anti-angiogene, antirestenotische, mikrotubuli-inhibierende, antimigrative oder antithrombotische Substanz.

Die Wirkstoff-Konzentration im Lösungsmittelgemisch beträgt vorzugsweise bis zu 100 mg/ml, bevorzugt 5 bis 100 mg Wirkstoff pro 1 ml Lösung Erfindungsgemäß können hohe Wirkstoffkonzentrationen von 45 bis 100 mg Wirkstoff pro ml Lösung eingesetzt werden, da die niedrige Verdampfungsgeschwindigkeit des zweiten Lösungsmittels eine Verwendung kleinerer Volumina der Beschichtungslösung gestattet.

Gemäß einer bevorzugten Ausführungsform enthält die zur Beschichtung der Substratoberfläche verwendete Lösung einen anti-proliferativen Wirkstoff, insbesondere Paclitaxel und/oder Rapamycin, bevorzugt in einem Anteil von zwischen etwa 5 bis 100 mg Wirkstoff pro 1 ml Lösung, besonders bevorzugt von 45 bis 100 mg/ml.

Darüber hinaus kann die Beschichtungslösung wenigstens eine darin gelöste Trägersubstanz für den Wirkstoff enthalten, die den Wirkstoff in der Beschichtung einbettet und/oder auf der Substratoberfläche hält.

Besonders bevorzugt besteht die Beschichtungslösung aus dem Wirkstoff und dem Gemisch aus dem ersten und zweiten Lösungsmittel.

Die Substratoberfläche kann auch vor dem Auftragen der Beschichtungslösung mit der Trägersubstanz beschichtet werden. In diesem Fall kann eine Trägersubstanz gewählt werden, die sich nicht in dem Lösungsmittelgemisch für den Wirkstoff löst.

Eine bevorzugte Trägersubstanz ist Schellack oder ein Schellackderivat, insbesondere wasserlöslicher Schellack. Schellack begünstigt außerdem die Freisetzung des Wirkstoffs in das Gewebe der Blutgefäße, insbesondere bei kurzen Kontaktzeiten. Eine weitere geeignete Trägersubstanz ist Polyvinylalkohol (PVA), der ebenfalls wasserlöslich ist.

PVA und wasserlöslicher Schellack werden bevorzugt als Trägersubstanz eingesetzt, die nicht in der Beschichtungslösung löslich ist und vor dem Auftragen der Beschichtungslösung auf die Substratoberfläche aufgetragen wird.

Als erstes Lösungsmittel für den Wirkstoff werden bevorzugt leichtflüchtige organische Lösungsmittel mit einem Dampfdruck von mindestens 40 hPa bei 20 °C verwendet. Beispiele für das erste Lösungsmittel sind Aceton, Ethylacetat, Methanol, Ethanol, Isopropanol, THF, Chloroform, Methylenchlorid sowie Mischungen davon.

Das zweite Lösungsmittel weist vorzugsweise einen Siedepunkt von mindestens 150 °C, besonders bevorzugt einen Siedepunkt im Bereich von 150 °C bis 200 °C auf. Der Dampfdruck des zweiten Lösungsmittels liegt bevorzugt im Bereich von 1 bis 5 hPa bei 20 °C.

Besonders bevorzugt weist das erste Lösungsmittel einen um mindestens den Faktor 10 höheren Dampfdruck (bei 20 °C) auf als das zweite Lösungsmittel.

Bevorzugt ist das zweite Lösungsmittel aus der aus Dimethylsulfoxid, Dimethylformamid und N, N-Dimethylacetamid und deren Mischungen bestehenden Gruppe ausgewählt. Besonders bevorzugt ist das zweite Lösungsmittel Dimethylsulfoxid (DMSO).

Wesentlich für die Durchführung des erfindungsgemäßen Verfahrens ist, dass der Wirkstoff sowohl im ersten als auch zweiten Lösungsmittel löslich ist. "Löslich" im Sinne der Erfindung bedeutet, dass der Wirkstoff bei 20 °C eine Löslichkeit von mindestens 100 mg/ml im jeweiligen Lösungsmittel aufweist.

Der Wirkstoff und das zweite Lösungsmittel sind in der Beschichtungslösung in einem Anteil enthalten, so dass der Anteil des Wirkstoffs über der Löslichkeitsgrenze des Wirkstoffs in dem Anteil des zweiten Lösungsmittels liegt. Dies bedeutet, dass die in der Beschichtungslösung enthaltene Wirkstoffmenge nicht allein von dem in der Lösung enthaltenen zweiten Lösungsmittel gelöst werden kann.

Bevorzugt ist der Wirkstoffanteil in der Beschichtungslösung mindestens etwa 10 % größer als die in dem Anteil des zweiten Lösungsmittels maximal lösliche Menge des Wirkstoffs. Besonders bevorzugt liegt der Wirkstoffanteil in der Beschichtungslösung im Bereich des 1,1 bis 5Fachen der maximalen Löslichkeit des Wirkstoffs in dem zweiten Lösungsmittel besonders bevorzugt im Bereich des 1,1 bis 2,5Fachen der maximalen Löslichkeit.

Der Anteil des ersten Lösungsmittels in der Beschichtungslösung soll mindestens dazu ausreichen, eine vollständige Lösung des Wirkstoffs bei der Beschichtungstemperatur, vorzugsweise bei 20 °C, zu bewirken. Über diesen Anteil hinaus kann die Menge des ersten Lösungsmittels beliebig erhöht werden.

Die Beschichtungslösung kann auf alle handelsüblichen Ballons von Ballonkathetern und/oder Stents zur Ausbildung einer wirkstoffhaltigen Beschichtung aufgetragen werden. Insbesondere eignen sich Stents und Ballons mit glatter Oberfläche, Ballons mit Furchen oder Poren und Ballons oder Stents mit strukturierten oder aufgerauten Oberflächen. Geeignet sind weiter Ballons von Kathetern, die mit Falten oder Flügeln versehen sind, insbesondere sogenannte Mehrfaltenballons.

Die Beschichtung der Ballonoberfläche kann im nicht expandierten Zustand oder in einem ganz oder teilweise expandierten Zustand erfolgen. Insbesondere mit Falten versehene Ballons werden vorzugsweise in einem wenigstens teilweise expandierten Zustand beschichtet.

Zum Auftragen der Beschichtungslösung auf die Substratoberfläche, insbesondere die Oberfläche des Ballons oder Stents, können die im Stand der Technik bekannten Verfahren verwendet werden, insbesondere Sprühbeschichten, Tauchbeschichten oder Beschichten mit Tintendruckverfahren wie Jetten.

Zur Verbesserung der Benetzung mit Beschichtungslösung kann die Substratoberfläche vor der Beschichtung einer Koronabehandlung oder einer Plasmabehandlung unterzogen werden.

Nach dem Auftragen der Beschichtungslösung wird die Substratoberfläche durch Entfernen der Lösungsmittel, vorzugweise im Luftstrom, getrocknet, so dass im Wesentlichen kein Lösungsmittel in der Beschichtung verbleibt. Soweit flüssige oder gelbildende Hilfsstoffe verwendet werden, die pharmazeutisch verträglich sind, können diese in der Beschichtung verbleiben.

Das Entfernen der Lösungsmittel wird so gesteuert, dass zunächst im Wesentlichen das erste Lösungsmittel aus der auf die Substratoberfläche aufgetragenen Beschichtungslösung entfernt wird, so dass eine Mischung aus dem Wirkstoff und dem zweiten Lösungsmittel auf der Substratoberfläche verbleibt, wahlweise zusammen mit einem geringen Anteil des ersten Lösungsmittels. Aus dieser Mischung dampft das zweite Lösungsmittel langsam ab, während der zunächst amorphe Wirkstoff auskristallisiert.

Die Steuerung der Verdampfungsgeschwindigkeit kann beispielsweise über die Substrattemperatur, die Umgebungstemperatur und/oder den Umgebungsdruck beim Entfernen der Lösungsmittel erfolgen, da der jeweilige Dampfdruck der Lösungsmittel druck- und temperaturabhängig ist. Aus dem Verhältnis der Dampfdrücke des ersten und zweiten Lösungsmittels folgt bei gegebener Temperatur und gegebenem Umgebungsdruck dann auch die Zusammensetzung der Dampfphase und der auf der Substratoberfläche vorhandenen Beschichtungslösung.

Bevorzugt wird das Entfernen der Lösungsmittel daher bei einer Temperatur im Bereich von 10 bis 40 °C, besonders bevorzugt bei Raumtemperatur (etwa 20-25 °C) durchgeführt, wobei wegen des um ein Vielfaches höheren Dampfdrucks das erste Lösungsmittel vor dem zweiten Lösungsmittel verdampft. Besonders bevorzugt werden die Umgebungsbedingungen so gewählt, dass der Partialdruck des ersten Lösungsmittels über der Beschichtungslösung um mindestens das 10fache höher ist als der Partialdruck des zweiten Lösungsmittels.

Beim gesteuerten Entfernen der Lösungsmittel entsteht somit ein Zwischenzustand der Beschichtung in Form einer amorphen, halbfesten Mischung des Wirkstoffs mit dem zweiten Lösungsmittel, die aufgrund der hohen Viskosität ortsfest auf der Substratoberfläche haftet. Wird das Entfernen der Lösungsmittel dagegen bei erhöhter Temperatur oder unter Vakuum durchgeführt, verdampft auch das zweite Lösungsmittel in relativ kurzer Zeit, wobei sich der Wirkstoff in amorpher Form aus der Beschichtungslösung abscheidet.

Über die Umgebungsbedingungen beim Entfernen der Lösungsmittel und das Verhältnis des Wirkstoffanteils zum Anteil des zweiten Lösungsmittels in der Lösung kann die Kristallmorphologie des Wirkstoffs in der nach Entfernen der Lösungsmittel erhaltenen Beschichtung eingestellt werden. Je länger der Wirkstoff in Kontakt mit dem zweiten Lösungsmittel steht, desto mehr Zeit wird für die Kristallbildung und das Kristallwachstum des Wirkstoffs zur Verfügung stehen. Während das zweite Lösungsmittel langsam aus der Beschichtung entfernt wird, tritt somit eine Umkristallisation des Wirkstoffs von einer amorphen Struktur in eine stabile Kristallphase ein, die fest auf der Substratoberfläche haftet.

Das Entfernen der Lösungsmittel wird bevorzugt so gesteuert, dass nach einem Zeitraum von bis zu etwa 15 min, bevorzugt etwa 5 bis 15 min, das erste Lösungsmittel entfernt und die Umkristallisation des Wirkstoffs von der amorphen in die kristalline Form abgeschlossen ist. Die vollständige Entfernung des zweiten Lösungsmittels aus der Beschichtung kann bis zu 24 Stunden dauern. Wahlweise kann die Umgebungstemperatur nach Abschluss der Umkristallisation auf bis zu 60 °C erhöht werden, um die Zeitdauer zur vollständigen Entfernung des zweiten Lösungsmittels aus der Beschichtung zu verkürzen.

Wird dagegen eine Beschichtungslösung verwendet, die nur das erste leichtflüchtige Lösungsmittel, beispielsweise Ethylacetat oder Ethanol, und den Wirkstoff enthält, entsteht durch die unmittelbare Verdunstung des Lösungsmittels eine klarlackartige, amorphe Beschichtung, aus der sich der Wirkstoff nicht mehr in eine kristalline schwerlösliche Form umbildet.

Nach Entfernen der Lösungsmittel wird erfindungsgemäß eine Beschichtung erhalten, die den Wirkstoff in kristalliner Form enthält.

Wahlweise kann die so erhaltene Beschichtung noch mit einer Schutzschicht, beispielsweise aus Schellack und/oder Polyvinylalkohol, versehen werden.

Im Anschluss an das Entfernen der Lösungsmittel, und wahlweise das Auftragen der Schutzschicht, kann das beschichtete Substrat mit bekannten Maßnahmen, beispielsweise durch Behandeln mit Ethylenoxid, sterilisiert und das Medizinprodukt steril verpackt werden.

Unter Verwendung der erfindungsgemäßen Beschichtungslösung lässt sich ein beschichtetes Medizinprodukt, beispielsweise ein Stent oder Ballonkatheter, mit einer festhaftenden Beschichtung aus einem kristallinen anti-proliferativen Wirkstoff wie Paclitaxel herstellen, aus der das Paclitaxel effektiv am Stenoseort freigesetzt und in kristalliner Form im Gewebe der Blutgefäßwand abgelagert werden kann. Wegen der verringerten Löslichkeit des kristallinen Wirkstoffs kann so die Freisetzungsrate im Gewebe verlängert werden.

Die Beschichtung auf der Substratoberfläche ist außerdem mechanisch stabil und löst sich beim Einführen des Katheters in die Blutgefäße nicht ab. Dadurch steht am Stenoseort eine hohe Wirkstoffkonzentration zur Verfügung. Außerdem bleibt das Freisetzungsprofil des Wirkstoffs auch bei längerer Lagerung des beschichteten Medizinprodukts über mehrere Wochen stabil.

Weitere Merkmale und Vorteile der Erfindung ergeben sich aus der nachfolgenden Beschreibung bevorzugter Ausführungsbeispiele unter Bezug auf die beigefügte Zeichnung. Die Ausführungsbeispiele sollen jedoch nicht in einem einschränkenden Sinn verstanden werden. In der Zeichnung zeigen:
- Figur 1 eine elektronenmikroskopische Aufnahme einer nach dem erfindungsgemäßen Verfahren erhaltenen Beschichtung aus kristallinem Paclitaxel in 1000facher Vergrößerung;
- Figur 2 eine elektronenmikroskopische Aufnahme der Beschichtung aus Figur 1 in einem Zwischenzustand nach dem Entfernen des ersten Lösungsmittels (1000fach vergrößert);
- Figur 3 eine elektronenmikroskopische Aufnahme einer Paclitaxel-Beschichtung auf wasserlöslichem Schellack in einer 5000fachen Vergrößerung, die nach dem erfindungsgemäßen Verfahren hergestellt wurde; und
- Figur 4 eine elektronenmikroskopische Aufnahme einer Paclitaxel-Beschichtung auf PVA in 1000facher Vergrößerung.

### Beispiel 1

Durch Eintrocknen einer Stammlösung von Paclitaxel in Ethylacetat mit definierter Konzentration wurden 10 mg Paclitaxel als Feststoff bereitgestellt. Das Paclitaxel wurde in Gemischen aus Ethylacetat mit unterschiedlichen Anteilen von Dimethylsulfoxid (DMSO) aufgenommen und geprüft, ob sich nach 72 h bei Raumtemperatur eine klare Lösung bildet.

In einem weiteren Ansatz wurde die maximale Löslichkeit von Paclitaxel in DMSO zu 400 mg/ml bestimmt.

Die Lösungsversuche zeigten, dass eine Zusammensetzung der Beschichtungslösung aus 10 mg Paclitaxel in 200 µl Ethylacetat und 12 µl DMSO bereits die gewünschte klare Lösung ergab. In dieser Lösung lag der Wirkstoffanteil bei etwa 833 mg/ml DMSO und betrug damit etwa das 2,1 Fache der maximalen Löslichkeit von Paclitaxel in DMSO.

### Beispiel 2

Es wurde eine Beschichtungslösung aus 10 mg Paclitaxel in 200 µl Ethylacetat und 22 µl DMSO hergestellt. In dieser Beschichtungslösung liegt der Wirkstoffanteil mit 454 mg Paclitaxel / ml DMSO um etwa 13,5 % über der maximalen Löslichkeit des Paclitaxel in DMSO. Der in der Lösung enthaltene DMSO-Anteil reicht somit nicht dazu aus, das im Lösungsmittelgemisch aus 200 µl Ethylacetat und 22 µl DMSO enthaltene Paclitaxel (10 mg) vollständig zu lösen.

Die Beschichtungslösung wurde auf ein Edelstahlplättchen aufgetragen und bei Raumtemperatur unter normalem Luftdruck belassen bis die Lösungsmittel vollständig abgedampft waren. Man erhielt eine Beschichtung aus Paclitaxel in kristalliner Struktur.

Eine Wiederholung des Beschichtungsversuchs mit der Lösung aus Beispiel 1 (10 mg Wirkstoff in 200 µl Ethylacetat und 12 µl DMSO) ergab ebenfalls eine kristalline Beschichtung von Paclitaxel.

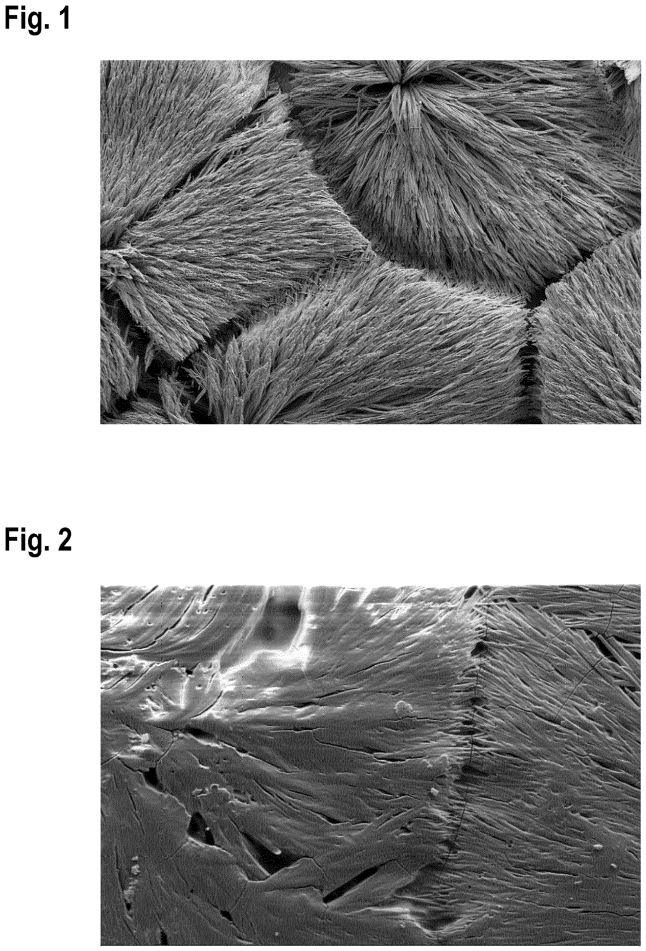

In Figur 1 ist eine elektronenmikroskopische Aufnahme der aus der Beschichtungslösung gemäß Beispiel 2 erhaltenen Beschichtung in 1000facher Vergrößerung gezeigt. Die nadelförmige Struktur der Paclitaxel-Kristalle ist in dieser Aufnahme deutlich zu erkennen.

Figur 2 zeigt eine elektronenmikroskopische Aufnahme der Beschichtung aus Figur 1 in einem Zwischenzustand nach dem Entfernen des ersten Lösungsmittels. Zu sehen ist, dass sich der zunächst amorphe Wirkstoff, der noch mit Anteilen des zweiten Lösungsmittels vermischt ist, in eine nadelförmige kristalline Struktur umwandelt.

### Beispiel 3

10 mg Paclitaxel wurden in 2000 µl Ethylacetat und 22 µl DMSO gelöst. Gegenüber Beispiel 2 war also die Menge des ersten, leicht flüchtigen Lösungsmittels verzehnfacht.

Die so erhaltene Beschichtungslösung wurde auf ein Edelstahlplättchen aufgetragen und bei Raumtemperatur unter normalem Luftdruck belassen bis die Lösungsmittel vollständig abgedampft waren. Man erhielt ebenfalls eine Beschichtung aus Paclitaxel in kristalliner Struktur. Damit konnte gezeigt werden, dass das zweite Lösungsmittel auch bei starker Verdünnung mit dem ersten leicht-flüchtigen Lösungsmittel in der Beschichtung vorhanden bleibt und nach dem Entfernen des ersten Lösungsmittels eine Umkristallisation des Wirkstoffs in eine kristalline Form bewirkt.

### Beispiele 4 und 5

In den folgenden Beispielen wurde die Zusammensetzung des ersten Lösungsmittels sowie der Anteil an DMSO weiter verändert, und es wurden Beschichtungslösungen mit folgenden Zusammensetzungen hergestellt:

### Beispiel 4:

10 mg Paclitaxel
+ 1 ml Methanol
+ 1 ml Ethanol
+ 5 µl DMSO

### Beispiel 5:

10 mg Paclitaxel
+ 1000 µl Chloroform
+100 µl Ethanol
+ 5 µl DMSO

Die so erhaltenen Beschichtungslösungen wurden wiederum auf ein Edelstahlplättchen aufgetragen und bei Raumtemperatur unter normalem Luftdruck belassen bis die Lösungsmittel vollständig abgedampft waren. Man erhielt eine Beschichtung aus Paclitaxel in kristalliner Struktur.

Auch in diesen Zusammensetzungen bleibt die geringe Menge DMSO eine ausreichend lange Zeit in der Beschichtung enthalten, um eine Umkristallisation des Paclitaxel in die kristalline Form herbeizuführen.

### Beispiel 6

Auf ein Edelstahlsubstrat wurde zunächst eine dünne Grundierung aus einem wasserlöslichen Schellack aufgetragen. Der wasserlösliche Schellack ist in Ethylacetat nicht löslich. Auf das mit wasserlöslichem Schellack behandelte Substrat wurde eine Beschichtungslösung gemäß Beispiel 7 aufgetragen.

Nach dem Abdampfen der Lösungsmittel bei Raumtemperatur wurde ein mit Paclitaxel in kristalliner Form beschichtetes Substrat erhalten.

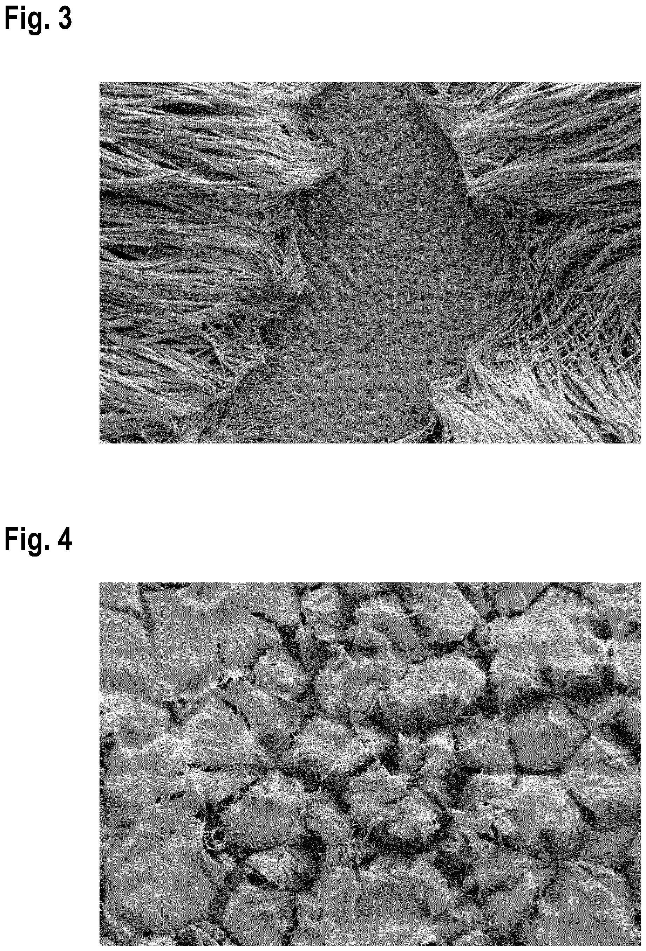

In Figur 3 ist eine elektronenmikroskopische Aufnahme der so erhaltenen Beschichtung gezeigt. Man erkennt die porige Grundierung aus wasserlöslichem Schellack und die darauf unvermischt aufliegenden Kristalle von Paclitaxel in Nadelform.

Beispiel 6 wurde unter Verwendung eines mit Polyvinylalkohol (PVA) behandelten Edelstahlsubstrats wiederholt. Die elektronenmikroskopische Aufnahme in Figur 4 zeigt die auf der PVA-Grundierung aufgewachsene Beschichtung aus nadelförmigen Paclitaxel-Kristallen in 200facher Vergrößerung.

### Beispiel 7

In einem weiteren Versuch wurden die gemäß Beispiel 6 erhaltenen Beschichtungen mit Paclitaxel in kristalliner Form mit wasserlöslichem Schellack und/oder mit einer wässrigen Lösung von Polyvinylalkohol unter Bildung einer Schutzschicht besprüht. In allen Fällen blieb die kristalline Form des Paclitaxel erhalten.

## Patentansprüche

1. Verfahren zur Herstellung eines Medizinprodukts mit Wirkstoffbeschichtung, wobei das Medizinprodukt ein Substrat mit einer Oberfläche und eine auf der Oberfläche aufgebrachte Beschichtung umfasst, die einen Wirkstoff enthält, wobei das Verfahren die folgenden Schritte umfasst:
Bereitstellen des Substrats mit der Oberfläche;
Bereitstellen einer Lösung des Wirkstoffs in einem Gemisch von wenigstens einem ersten und einem zweiten Lösungsmittel, wobei der Wirkstoff sowohl in dem ersten als auch dem zweiten Lösungsmittel löslich ist;
Auftragen der Lösung auf wenigstens einen Teil der Oberfläche des Substrats; und
Entfernen der Lösungsmittel unter Bildung einer den Wirkstoff enthaltenden Beschichtung auf der Oberfläche;
wobei das erste Lösungsmittel einen Siedepunkt von unter 100 °C und das zweite Lösungsmittel einen Siedepunkt von über 130 °C aufweist und die Lösung den Wirkstoff und das zweite Lösungsmittel in einem Anteil enthält, so dass der Anteil des Wirkstoffs über der Löslichkeitsgrenze des Wirkstoffs in dem Anteil des zweiten Lösungsmittels liegt, und wobei das Entfernen der Lösungsmittel so gesteuert wird, dass nach Entfernen der Lösungsmittel eine Beschichtung erhalten wird, die den Wirkstoff in kristalliner Form enthält.

2. Verfahren nach Anspruch 1, **dadurch gekennzeichnet, dass** als Wirkstoff ein anti-proliferativer Wirkstoff, insbesondere Paclitaxel oder Rapamycin, verwendet wird.

3. Verfahren nach Anspruch 1 oder 2, **dadurch gekennzeichnet, dass** das erste Lösungsmittel einen Dampfdruck bei 20 °C von mindestens 40 hPa aufweist.

4. Verfahren nach Anspruch 3, **dadurch gekennzeichnet, dass** das erste Lösungsmittel aus der aus Aceton, Ethylacetat, Methanol, Ethanol, Isopropanol, Tetrahydrofuran, Chloroform, Ethylenchlorid und deren Mischungen bestehenden Gruppe ausgewählt wird.

5. Verfahren nach einem der Ansprüche 1 bis 4, **dadurch gekennzeichnet, dass** das zweite Lösungsmittel einen Dampfdruck bei 20 °C im Bereich von 1 - 5 hPa aufweist.

6. Verfahren nach Anspruch 5, **dadurch gekennzeichnet, dass** das zweite Lösungsmittel aus der aus Dimethylsulfoxid, Dimethylformamid und N, N-Dimethylacetamid und deren Mischungen bestehenden Gruppe ausgewählt ist.

7. Verfahren nach einem der Ansprüche 1 bis 6, **dadurch gekennzeichnet, dass** das erste Lösungsmittel einen um mindestens das Zehnfache höheren Dampfdruck bei 20 °C aufweist als das zweite Lösungsmittel.

8. Verfahren nach einem der Ansprüche 1 bis 7, **dadurch gekennzeichnet, dass** der Wirkstoffanteil in der Beschichtungslösung mindestens etwa 10 % größer als die in dem Anteil des zweiten Lösungsmittels maximal lösliche Menge des Wirkstoffs ist.

9. Verfahren nach Anspruch 8, **dadurch gekennzeichnet, dass** der Wirkstoffanteil in der Beschichtungslösung im Bereich des 1,1 bis 5Fachen der maximalen Löslichkeit des Wirkstoffs in dem Anteil des zweiten Lösungsmittels liegt.

10. Verfahren nach Anspruch 9, **dadurch gekennzeichnet, dass** der Wirkstoffanteil im Bereich des 1,1 bis 2,5Fachen der maximalen Löslichkeit des Wirkstoffs in dem Anteil des zweiten Lösungsmittels liegt.

11. Verfahren nach einem der vorhergehenden Ansprüche, **dadurch gekennzeichnet, dass** zunächst im Wesentlichen das erste Lösungsmittel und danach das zweite Lösungsmittel von der Substratoberfläche entfernt wird.

12. Verfahren nach einem der vorhergehenden Ansprüche, **dadurch gekennzeichnet, dass** die Lösungsmittel unter Normaldruck bei einer Temperatur im Bereich von 10 - 40 °C entfernt werden.

13. Verfahren nach Anspruch 12, **dadurch gekennzeichnet, dass** die Temperatur im Bereich von 20 - 30 °C liegt.

14. Verfahren nach einem der vorhergehenden Ansprüche, **dadurch gekennzeichnet, dass** die Umgebungsbedingungen mit Bezug auf Druck und Temperatur beim Entfernen der Lösungsmittel so eingestellt werden, dass der Partialdruck des ersten Lösungsmittels der Beschichtungslösung um mindestens das Zehnfache höher ist als der Partialdruck des zweiten Lösungsmittels.

15. Verfahren nach einem der vorhergehenden Ansprüche, **dadurch gekennzeichnet, dass** als Wirkstoff Paclitaxel verwendet wird und der Wirkstoff in der Beschichtung in Form von nadelförmigen Kristallen vorliegt.

16. Verfahren nach einem der vorhergehenden Ansprüche, **dadurch gekennzeichnet, dass** das Substrat ein Stent oder Ballon eines Ballonkatheters ist.

17. Verfahren nach einem der vorhergehenden Ansprüche, **dadurch gekennzeichnet, dass** auf die den Wirkstoff in kristalliner Form enthaltende Beschichtung eine Schutzschicht aufgebracht wird.

## Claims

1. A method of manufacturing a medical product having an active ingredient coating, the medical product comprising a substrate having a surface and a coating applied to the surface, the coating containing an active ingredient, the method comprising the following steps:
providing the substrate having the surface;
providing a solution of the active ingredient in a mixture of at least a first and a second solvent, the active ingredient being soluble in both the first and the second solvent;
applying the solution to at least part of the surface of the substrate; and
removing the solvents to form a coating on the surface, the coating containing the active ingredient;
the first solvent having a boiling point below 100°C and the second solvent having a boiling point above 130°C, and the solution containing the active ingredient and the second solvent in a proportion such that the proportion of the active ingredient is above the solubility limit of the active ingredient in the proportion of the second solvent, and the removing of the solvents being controlled such that after removing the solvents a coating is obtained which contains the active ingredient in a crystalline form.

2. The method according to claim 1, **characterized in that** the active ingredient used is an anti-proliferative active ingredient, in particular paclitaxel or rapamycin.

3. The method according to claim 1 or 2, **characterized in that** the first solvent has a vapor pressure of at least 40 hPa at 20°C.

4. The method according to claim 3, **characterized in that** the first solvent is selected from the group consisting of acetone, ethyl acetate, methanol, ethanol, isopropanol, tetrahydrofuran, chloroform, ethylene chloride and mixtures thereof.

5. The method according to any of claims 1 to 4, **characterized in that** the second solvent has a vapor pressure in the range of from 1 to 5 hPa at 20°C.

6. The method according to claim 5, **characterized in that** the second solvent is selected from the group consisting of dimethyl sulfoxide, dimethyl formamide and N, N-dimethylacetamide and mixtures thereof.

7. The method according to any of claims 1 to 6, **characterized in that** the first solvent has a vapor pressure at 20°C that is at least ten times higher than that of the second solvent.

8. The method according to any of claims 1 to 7, **characterized in that** the active ingredient content in the coating solution is at least about 10% higher than the maximum soluble amount of the active ingredient in the proportion of the second solvent.

9. The method according to claim 8, **characterized in that** the active ingredient content in the coating solution is in the range of from 1.1 to 5 times the maximum solubility of the active ingredient in the proportion of the second solvent.

10. The method according to claim 9, **characterized in that** the active ingredient content is in the range of from 1.1 to 2.5 times the maximum solubility of the active ingredient in the proportion of the second solvent.

11. The method according to any of the preceding claims, **characterized in that** initially essentially the first solvent and thereafter the second solvent is removed from the substrate surface.

12. The method according to any of the preceding claims, **characterized in that** the solvents are removed under normal pressure at a temperature in the range of 10 to 40°C.

13. The method according to claim 12, **characterized in that** the temperature is in the range of 20 to 30°C.

14. The method according to any of the preceding claims, **characterized in that** when removing the solvents, the ambient conditions with respect to pressure and temperature are adjusted such that the partial pressure of the first solvent of the coating solution is at least ten times higher than the partial pressure of the second solvent.

15. The method according to any of the preceding claims, **characterized in that** paclitaxel is used as the active ingredient and the active ingredient is present in the coating in the form of acicular crystals.

16. The method according to any of the preceding claims, **characterized in that** the substrate is a stent or a balloon of a balloon catheter.

17. The method according to any of the preceding claims, **characterized in that** a protective layer is applied to the coating containing the active ingredient in crystalline form.

## Revendications

1. Procédé de fabrication d'un produit médical présentant un revêtement de matière active, le produit médical comprenant un substrat qui présente une surface et un revêtement qui est appliqué sur la surface et qui contient une matière active, le procédé comprenant les étapes suivantes :
fournir le substrat qui présente la surface ;
fournir une solution de la matière active dans un mélange d'au moins un premier et un deuxième solvant, la matière active étant soluble tant dans le premier que dans le deuxième solvant ;
appliquer la solution au moins sur une partie de la surface du substrat ; et
retirer le solvant en formant un revêtement contenant la matière active sur la surface ;
le premier solvant présentant un point d'ébullition inférieur à 100°C, et le deuxième solvant présentant un point d'ébullition supérieur à 130°C, et la solution contenant la matière active et le deuxième solvant dans une telle part que la part de la matière active est supérieure à la limite de solubilité de la matière active dans la part du deuxième solvant, et le retrait du solvant étant commandé de telle sorte qu'un revêtement contenant la matière active dans une forme cristalline est obtenu après le retrait des solvants.

2. Procédé selon la revendication 1, **caractérisé en ce qu'**une matière active antiproliférative, en particulier le paclitaxel ou la rapamycine est utilisé(e) en tant que matière active.

3. Procédé selon la revendication 1 ou 2, **caractérisé en ce que** le premier solvant présente à une température de 20°C une pression de vapeur d'au moins 40 hPa.

4. Procédé selon la revendication 3, **caractérisé en ce que** le premier solvant est choisi dans le groupe constitué d'acétone, d'acétate d'éthyle, de méthanol, d'éthanol, d'isopropanol, de tétrahydrofurane, de chloroforme, de chlorure d'éthylène et de leurs mélanges.

5. Procédé selon l'une des revendications 1 à 4, **caractérisé en ce que** le deuxième solvant présente à une température de 20°C une pression de vapeur dans une plage de 1 à 5 hPa.

6. Procédé selon la revendication 5, **caractérisé en ce que** le deuxième solvant est choisi dans le groupe constitué de diméthylsulfoxyde, de diméthylformamide et de N,N-diméthylacétamide et de leurs mélanges.

7. Procédé selon l'une des revendications 1 à 6, **caractérisé en ce que** le premier solvant présente à une température de 20°C une pression de vapeur au moins dix fois plus élevée que le deuxième solvant.

8. Procédé selon l'une des revendications 1 à 7, **caractérisé en ce que** la part de matière active dans la solution de revêtement est supérieure d'au moins 10 % à la quantité soluble maximale de la matière active dans la part du deuxième solvant.

9. Procédé selon la revendication 8, **caractérisé en ce que** la part de matière active dans la solution de revêtement est dans une plage de 1,1 à 5 fois la solubilité maximale de la matière active dans la part du deuxième solvant.

10. Procédé selon la revendication 9, **caractérisé en ce que** la part de matière active est dans une plage de 1,1 à 2,5 fois la solubilité maximale de la matière active dans la part du deuxième solvant.

11. Procédé selon l'une des revendications précédentes, **caractérisé en ce que** tout d'abord sensiblement le premier solvant et ensuite le deuxième solvant est retiré de la surface du substrat.

12. Procédé selon l'une des revendications précédentes, **caractérisé en ce que** les solvants sont retirés sous pression normale à une température dans une plage de 10 à 40°C.

13. Procédé selon la revendication 12, **caractérisé en ce que** la température est dans une plage de 20 à 30°C.

14. Procédé selon l'une des revendications précédentes, **caractérisé en ce que** les conditions ambiantes concernant la pression et la température sont ajustées lors du retrait des solvants de telle sorte que la pression partielle du premier solvant de la solution de revêtement est au moins 10 fois plus élevée que la pression partielle du deuxième solvant.

15. Procédé selon l'une des revendications précédentes, **caractérisé en ce que** le paclitaxel est utilisé en tant que matière active et **en ce que** la matière active est présente dans le revêtement sous forme de cristaux en aiguilles.

16. Procédé selon l'une des revendications précédentes, **caractérisé en ce que** le substrat est un stent ou un ballonnet d'un cathéter à ballonnet.

17. Procédé selon l'une des revendications précédentes, **caractérisé en ce qu'**une couche de protection est appliquée sur le revêtement contenant la matière active en forme cristalline.
